# EUROPEAN PATENT APPLICATION

(11) **EP 2 377 535 A1**
(43) Date of publication of application: **19.10.2011**
(21) Application number: 09838477.9
(22) Date of filing: 28.08.2009
(51) Int. Cl.: A61K 31/4184, A61K 31/14, A61K 47/32, A61K 47/10, A61K 9/48, A61K 9/20, A61K 9/08, A61P 25/28

(54) **METHOD AND COMPOSITION FOR TREATING MULTIPLE SCLEROSIS (VARIANTS)**

(30) Priority: 15.01.2009 RU 2009101125
(71) Applicant: Averin, Konstantin Mihailovich, Rostovskaya obl. 346900 (RU); Solodunov, Yuriy Yuryevich, Rostov-na-Donu 344048 (RU); Stradomskiy, Boris Vitalyevich, Rostov-na-Donu, 344052 (RU); Vilkov, Gennady Alexeevich, Rostov-na-Donu 344019 (RU)
(72) Inventor: Stradomskiy, Boris Vitalyevich, Rostov-na-Donu 344052 (RU); Vilkov, Gennady Alexeevich, Rostov-na-Donu 344019 (RU)
(74) Representative: Einsel, Martin
(86) International application number: PCT/RU2009/000433
(87) International publication number: WO 2010/082863

(57) **Abstract**

The invention relates to psychoneurology, more particularly to a method for treating multiple sclerosis that involves the use of a pharmaceutical composition containing 1,3-diethylbenzimidazole triiodide as the active compound. The composition (in solid or liquid form) for treating multiple sclerosis contains 1,3-diethylbenzimidazole triiodide and medicinal low-molecular polyvinylpyrrolidone and also, in the liquid form, ethyl alcohol in the following component ratio: liquid form - 1.5-10.0 mass% 1,3-diethylbenzimidazole triiodide, 6.0-30.0 mass% medicinal low-molecular polyvinylpyrrolidone and 60.0-92.5 mass% 95% ethyl alcohol; solid form - 10.0-25.0 mass% 1,3-diethylbenzimidazole triiodide and 75.0-90.0 mass% medicinal low-molecular polyvinylpyrrolidone.

## Description

### FIELD OF THE INVENTION

The invention relates to psychoneurology, particularly, to drugs for treating multiple sclerosis.

### BACKGROUND OF THE INVENTION

A prior art medication, Betaferon, a recombinant human Beta-1b interferon, is used for treating multiple sclerosis (Mashkovsky, M.D., "Drugs," Novaya Volna, Moscow, 2003, pp. 324-325). Betaferon is the only drug known today that has proved to be effective in a long-term (five years) placebo-controlled study (Totolian, N.A., "Betaferon in Multiple Sclerosis Treatment: An Efficiency Proof Standard." In: Journal of Neurology and Psychiatry, No. 7, 2005, pp. 68-71 ).

This medication is disadvantageous because of its relatively low efficiency in use, in particular, the frequency of acute conditions occurring in the disease and their severity are reduced by 30% only, and it causes side effects such as intensified depressive symptomatology (Sokolova, E.S., Totolian, N.A., Klimenko, V.M., "Psychoemotional Changes under the Effect of Betaferon Treatment of Multiple Sclerosis Patients." In: Stress and Behavior Seventh Interdisciplinary Conference on Biological Psychiatry, Novosibirsk, 2003), and very high costs of Betaferon pharmacotherapy.

Also in recent years, Copaxon, an acetate glatiramer has been used with significant success to treat multiple sclerosis (Zavalishin, I.A., Schwartz, G.Y., "Copaxon in Multiple Sclerosis Treatment (Collection of Articles)," Miklosh, Moscow, 2007, pp. 5-8). The high costs is certainly a disadvantage of Copaxon pharmacotherapy.

Benzimidazole derivatives, compositions containing the same, and production and application thereof disclosed in Patent No. 2007112501 are the closest prior art of the present invention in the combination of essential characteristics of the method and composition serving the same purpose. The compounds of the formula given in the patent, their pharmaceutically acceptable salts, their diastereomers, enantiomers or mixtures thereof are used for preparing a drug suitable for treating multiple sclerosis. The compounds of the general formula given in the patent are not, however, derivatives of the benzimidazole cation, for which reason they cannot form pharmacologically very active complex compounds with the imidazole ring of these compounds.

### SUMMARY OF THE INVENTION

It is an object and purpose of this invention to develop new preparations displaying a high pharmacological activity in treating multiple sclerosis, having a high therapeutic efficiency, lowering undesirable side effects, and reducing treatment costs.

The technical result that can be achieved by using the invention consists in developing a composition of a high pharmacological activity for treating multiple sclerosis by adding thereto a complex compound of active iodine and a diethyl derivative of benzimidazole cation, in particular, 1,3-diethylbenzimidazole triiodide as the active compound, and also developing a new highly effective method for treating multiple sclerosis.

The technical result is achieved in a method for treating multiple sclerosis by using a pharmaceutical composition comprising 1,3-diethylbenzimidazole triiodide as the active compound.

The technical result is also achieved by a composition (in solid or liquid form) for treating multiple sclerosis that comprises 1,3-diethylbenzimidazole triiodide of high pharmacological activity as the active compound, medicinal low-molecular polyvinylpyrrolidone that is a solubilizer and stabilizer of the active compound, and, additionally in the liquid form, a solvent (ethyl alcohol), in the following proportions of the components, in mass%:

| Liquid form: | | |
|---|---|---|
| | 1,3-diethylbenzimidazole triiodide | 1.5-10.0 |
| | Medicinal low-molecular polyvinylpyrrolidone | 6.0-30.0 |
| | Ethyl alcohol, 95% | 60.0-92.5 |

| Solid form: | | |
|---|---|---|
| | 1,3-diethylbenzimidazole triiodide | 10.0-25.0 |
| | Medicinal low-molecular polyvinylpyrrolidone | 75.0-90.0 |

### DESCRIPTION OF THE PREFERRED EMBODIMENTS OF THE INVENTION

The selected concentration ranges of the composition components are based on the following considerations. A concentration of the active compound - 1,3-diethylbenzimidazole triiodide - below 1.5 mass% lowers the therapeutic activity of the composition. A concentration of 1,3-diethylbenzimidazole triiodide above 25.0 mass% is likely to cause an irreversible loss of active compound solubility, reducing the therapeutic activity of the composition as well. A concentration of polyvinylpyrrolidone below 6.0 mass% does not guarantee the required solubility of 1,3-diethylbenzimidazole triiodide. The concentration of the solvent (ethyl alcohol) and its proportion in the composition depends on the consistency of the composition (solid drug or solution) required in each particular case. When the composition is produced as a solid drug, there is no need for solvents, while polyvinylpyrrolidone is essential to make the active compound soluble in the digestive tract. The concentration of solvents above 92.5 mass% lowers the content of the active compound - 1,3-diethylbenzimidazole triiodide - in mass% in the composition and, therefore, reduces the therapeutic activity of the composition.

The method for preparing a composition for treating multiple sclerosis may be carried out, provided that the above proportions are maintained, as follows: 1,3-diethylbenzimidazole triiodide (Pharmacopoeia Abstract 42-0609-6576-05) is used as the active compound of the composition prepared and has a prevailing effect on the quality and quantitative proportions of the composition. When the composition is prepared in liquid form with 95% ethyl alcohol (Pharmacopoeia Abstract 42-3072-00), white powder of medicinal low-molecular polyvinylpyrrolidone (Pharmacopoeia Abstract 42-03-84-2668-02) used as a solubilizer and stabilizer of the active compound is dissolved in 95% ethyl alcohol. At the next step, finely ground dark-brown crystals of 1,3-diethylbenzimidazole triiodide are dissolved in the solution obtained.

In order to prepare solid forms of the composition, finely ground crystals of 1,3-diethylbenzimidazole triiodide are mixed mechanically with white powder of medicinal low-molecular polyvinylpyrrolidone. The mixture obtained is used to made solid drug forms (tablets and capsules).

The method for preparing the composition for treating multiple sclerosis is safe to persons involved in the preparation process.

The possibility of the invention being carried out is supported by the following examples:

### Example 1

16.0 mass% of medicinal low-molecular polyvinylpyrrolidone (m.m. 12,600 D) was dissolved in 80.0 mass% of ethyl alcohol at a temperature of 55±5°C. The resultant solution was used to dissolve 4.0 mass% of 1 ,3-diethylbenzimidazole triiodide crystals. The composition (a solution for peroral administration) thus obtained was suitable for treating multiple sclerosis.

### Example 2

6.0 mass% of medicinal low-molecular polyvinylpyrrolidone (m.m. 12,600 D) was dissolved in 92.0 mass% of ethyl alcohol at a temperature of 55±5°C. The resultant solution was used to dissolve 2.0 mass% of 1,3-diethylbenzimidazole triiodide crystals. The composition (a solution for peroral administration) thus obtained was suitable for treating multiple sclerosis.

### Example 3

75.0 mass% of medicinal low-molecular polyvinylpyrrolidone (m.m. 12,600 D) was mixed mechanically with 25.0 mass% of finely ground 1,3-diethylbenzimidazole triiodide crystals. Measured doses of the resultant mixture were packed into gelatin capsules. The capsules for peroral administration were a solid drug form suitable for treating multiple sclerosis.

### Example 4

90.0 mass% of medicinal low-molecular polyvinylpyrrolidone (m.m. 12,600 D) was mixed mechanically with 10.0 mass% of finely ground 1,3-diethylbenzimidazole triiodide crystals. Measured doses of the resultant mixture were pressed into tablets, a solid drug form for peroral administration suitable for treating multiple sclerosis.

The clinical efficiency of the drug was supported by the administration of the claimed composition for treating multiple sclerosis patients. The composition variants described in the above examples were taken by the patients examined twice a day perorally in quantities containing a dose equivalent to 40 mg of the active compound.

### Patient B., b. 1979

The patient showed signs of the disease in September 2003, in particular, weakness in the legs and deteriorating eyesight of the right eye.

Neurological status: Broad sweeping horizontal nystagmus, high knee jerks, bilateral Babinski symptom. Pyramidal type reflexes, instability in the Romberg station.

MRT of November 20, 2003. Summary: The MRT shows signs of multiple demyelination foci in an acute stage and atrophic changes in the cerebral cortex.

Beginning in 2003, pharmacotherapy was given by the composition obtained in Example 1. Six weeks later, the condition started improving, and significant improvement was recorded three months later.

MRT of January 13, 2005. Summary: The MRT shows signs of a demyelinating disease of the brain.

MRT of July 23, 2006. Summary: The MRT shows signs of multiple sclerosis with elements of atrophy of the cerebral hemispheres. The condition stabilized in comparison with the MRT of January 13, 2005.

### Patient K., b. 1981

The patient showed signs of the disease in September 2003, in particular, weakness, numbness of the right limbs, and double vision looking sideways.

Neurological status: Horizontal and vertical nystagmus, paresis of craniocerebral nerves VI and VII, dynamic and static ataxia, high tendon reflexes, swaying in the Romberg station, and bilateral Babinski symptom.

The patient received treatment at the clinic of the neurology chair of Rostov State University of Medicine between February 17, 2006 and February 27, 2006. Clinical diagnosis: multiple sclerosis in cerebrospinal form, acute stage, pyramidal cerebellar syndrome. Treatment included Contrical, Hemodez, Pyracetam, vitamins A, E, and B₆, Solumedrol, and Carbamazepine.

Discharged with the disability status.

MRT of September 10, 2004. Summary: The MRT shows signs of a demyelinating disease of the brain.

MRT of February 27, 2006. Summary: The MRT shows signs of a demyelinating disease. New trunk and hemisphere foci have been detected.

The patient took the composition of Example 2 since March 2006. Reduction in the symptoms was recorded three months later, and the patient could take up a job.

### Patient K., b. 1971

The patient showed signs of the disease in May 2002, in particular, dizziness and deteriorating eyesight. The last acute condition occurred in summer 2005, when numbness of the right arm and blurred vision were recorded.

The patient was hospitalized at the clinic of nervous diseases and neurosurgery of Rostov State University of Medicine between November 28, 2005 and December 8, 2005, and at the neurology department of Hospital 1 in Rostov on Don between February 20, 2007 and February 28, 2007, and also between December 26, 2007 and January 10, 2008, diagnosed with multiple sclerosis, relapsing remittent course, acute condition, pronounced pyramidal cerebellar syndrome, functional disorder of the pelvic organs, and asthenocephalic syndrome. The treatment was traditional Copaxon since March 2006.

MRT of November 24, 2005. Summary: The MRT shows signs of multiple sclerosis.

Treatment began in March 2008 with the composition of Example 3 against the background of the acute condition caused by Copaxon. The patient's condition improved significantly within two to three months, and he could resume working. No acute conditions have since been recorded against the background of composition pharmacotherapy.

### Patient S., b. 1957

The patient was hospitalized at the neurology department of the Railroad Hospital and Hospital 6 in Rostov on Don in 2002, 2003, and 2005, diagnosed with multiple sclerosis, cerebrospinal form, progressive course, and tetraparesis in the dynamic deterioration stage. She was in disability Group II.

MRT of May 14, 2002. Summary: The MRT shows signs of demyelinating damage to the brain.

MRT of May 13, 2005. Summary: The MRT shows signs of a demyelinating disease. The number of foci remained stable, and there were progressing atrophic changes.

The patient has received therapeutic treatment with the composition of Example 4 since 2005. No acute conditions have been recorded since the start of treatment with the composition over the observation period, and the neurological status is stable.

### Patient Sh., b. 1981

The patient showed signs of the disease in September 2005, in particular, violent headaches, and rapid deterioration of eyesight of the right eye from 1.0 to 0.1.

Neurological status: Asthenocephalic syndrome, higher nervous activity - emotionally labile, asthenized, and anxious. Craniocerebral nerves - eyesight deterioration in the right eye, bilateral nystagmus, swaying in the Romberg station, coordination tests performed uncertainly, with intension and misses.

The patient was hospitalized for treatment at the neurology department of Hospital 6 in Rostov on Don between July 13, 2006 and August 4, 2006. She received parenteral treatment with Pentoxyphylline, Mexidole, Riboxin, Actovegin, Pyracetam, and Mildronate, and, enterally, Atarax, Berlithione, and FTL. The neurological status upon discharge did not differ from that recorded upon admission.

MRT of the brain of October 20, 2005. Summary: The MRT show signs of multifocal damage to the white matter of the brain.

MRT of the brain of March 28, 2006. Summary: There has been regress in the right peduncle of the brain and a new focus in the elevation of the *corpus callosum.*

Treatment with the composition prepared as described in Example 1 was started in September 2006. Three months later, a reduction in the symptoms was recorded and eyesight recovered in the right eye.

The MRT of May 31, 2007 showed a reduction in the process as compared to the MRT of March 28, 2006.

The MRT of May 15, 2008 showed signs of multiple sclerosis and a reduction in the process.

At present, the patient has no complaints and can work now.

The composition of the claimed makeup can be obtained by a technological method. It can be used as a drug for treating multiple sclerosis in medicine.

## Claims

1. A method for treating multiple sclerosis by using a pharmaceutical composition containing 1,3-diethylbenzimidazole triiodide as the active compound.

2. A composition for treating multiple sclerosis comprising 1,3-diethylbenzimidazole triiodide as the active compound, medicinal low-molecular polyvinylpyrrolidone as the solubilizer and stabilizer of the active compound, and 95% ethyl alcohol as the solvent in the following proportions of the components, in mass%:
| | |
|---|---|
| 1,3-diethylbenzimidazole triiodide | 1.5-10.0 |
| Medicinal low-molecular polyvinylpyrrolidone | 6.0-30.0 |
| Ethyl alcohol | 60.0-92.5. |

3. A composition for treating multiple sclerosis comprising 1,3-diethylbenzimidazole triiodide as the active compound and medicinal low-molecular polyvinylpyrrolidone as the solubilizer and stabilizer of the active compound in the following proportions of the components, in mass%:
| | |
|---|---|
| 1,3-diethylbenzimidazole triiodide | 10.0-25.0 |
| Medicinal low-molecular polyvinylpyrrolidone | 75.0-90.0. |
